# EUROPÄISCHE PATENTSCHRIFT

(11) **EP 3 682 794 B1**
(45) Veröffentlichungstag und Bekanntmachung des Hinweises auf die Patenterteilung: **08.03.2023**
(21) Anmeldenummer: 20151865.1
(22) Anmeldetag: 15.01.2020
(51) Int. Cl.: A61B 3/103, A61B 3/10

(54) **VERFAHREN UND SEHPRÜFSYSTEM ZUM ÜBERPRÜFEN DER AUGEN**
VISION TESTING SYSTEM AND METHOD FOR TESTING EYES
PROCÉDÉ ET SYSTÈME D'EXAMEN DE LA VUE DESTINÉS AU CONTRÔLE DES YEUX

(30) Priorität: 21.01.2019 DE 102019101409
(43) Veröffentlichungstag der Anmeldung: 22.07.2020
(73) Patentinhaber: Oculus Optikgeräte GmbH, 35582 Wetzlar (DE)
(72) Erfinder: STEINMÜLLER, Andreas, 35435 Wettenberg (DE)
(74) Vertreter: advotec.

(56) Entgegenhaltungen:
- EP-A1- 1 138 257
- EP-A1- 2 653 095
- EP-A1- 3 210 526
- EP-A1- 3 222 204
- DE-A1-102017 210 577
- JP-A- H0 531 073
- US-A1- 2003 142 271
- US-A1- 2006 209 256
- US-A1- 2012 197 102
- US-A1- 2013 100 408

## Beschreibung

Die Erfindung betrifft ein Verfahren zum Überprüfen der Augen eines Probanden mit den Merkmalen des Anspruchs 1 und ein Sehprüfsystem mit den Merkmalen des Anspruchs 10.

Refraktive Messvorrichtungen sind hinreichend bekannt und dienen regelmäßig zur Bestimmung eines Refraktionswertes eines Auges eines Probanden. Der Refraktionswert kann zur Beurteilung einer optischen Fehlsichtigkeit des Auges herangezogen werden. Eine häufige Form der Fehlsichtigkeit ist die Kurzsichtigkeit, bei der eine Bildebene vor der Netzhaut liegt, wodurch ein unscharfer Seheindruck entsteht. Bei der Kurzsichtigkeit (Myopie) wird zwischen verschiedenen Formen unterschieden. Bei der sogenannten Achsenmyopie ist eine axiale Länge des Auges erhöht. Bei der Refraktionsmyopie liegt eine erhöhte Brechkraft der refraktiven Teile des Auges, beispielsweise Hornhaut oder Linse, vor. Die erhöhte Brechkraft kann beispielsweise durch eine verstärkte Krümmung einer refraktiven Fläche, wie der Hornhaut, verursacht sein. Auch kann ein Brechungsindex der Linse verändert sein, sodass sich hieraus eine erhöhte Brechkraft ergibt. Kurzsichtigkeit kann sich bereits im Kindesalter durch verschiedene Umgebungsfaktoren, wie Sehaufgaben im Nahbereich des Auges, entwickeln oder mit dem fortschreitenden Alter entstehen, beispielsweise durch Nachlassen der Akkommodationsfähigkeit des Ziliarmuskels mit der Linse. Neben Medikamenteneinnahme können auch Krankheiten, wie Diabetes mellitus oder eine genetische Disposition eine Ursache für Kurzsichtigkeit sein.

Mit einer refraktiven Messvorrichtung kann ein Refraktionswert in Dioptrien (dpt) vergleichsweise einfach objektiv bestimmt werden. So ist aus der DE 102 006 017 389 A1 ein Sehprüfsystem bekannt, welches aus einer refraktiven Messvorrichtung und einer topografischen Messvorrichtung gebildet ist. Bei der refraktiven Messvorrichtung handelt es sich hier um ein Autorefraktometer, welches mit der topografischen Messvorrichtung, die in Art eines Keratometers ausgebildet ist, kombiniert ist. Mit dem Keratometer kann eine Topografie der Hornhaut bestimmt werden, wodurch Informationen über eine mögliche Ursache einer Kurzsichtigkeit, beispielsweise einem eventuell vorhandenen Katarakt, gewonnen werden können.

Dennoch ist die Beurteilung der so erhaltenen Refraktions- und Topografiedaten schwierig, da die Refraktionsmessung fehlerhaft sein kann, und auch andere Einflussfaktoren für bestimmte Refraktionswerte ursächlich sein können. Eine fehlerhafte Refraktionsmessung kann sich beispielsweise dadurch ergeben, dass der Proband während der Messung mit dem Auge im Nahbereich akkomodiert. Um dies auszuschließen kann der Ziliarmuskel medikamentös gelähmt werden (Zykloplegie), was jedoch zeitaufwendig und für den Probanden unangenehm ist.

Aus der US 2012/197102 A1 ist ein für Augenoperationen nutzbares Mikroskop bekannt, welches für Eingriffe zur Implementierung von Intraokularlinsen verwendet werden soll. In den Strahlengang des Mikroskops ist unter anderem eine erste Messvorrichtung zur Messung einer Achslänge eines Auges, eine zweite Messvorrichtung zur Messung einer Krümmung der Hornhaut des Auges und eine dritte Messvorrichtung zur Messung einer objektiven Refraktion des Auges integriert. Weiter ist eine Verarbeitungseinrichtung vorgesehen, mit der Daten der jeweiligen Messvorrichtungen verarbeitet werden können. Insbesondere kann auch ein Vergleich von Messdaten von vor und nach einer Operation des Auges durchgeführt werden.

Die EP 3 222 204 A1 beschreibt ein Messsystem bzw. ein Verfahren zur Augenuntersuchung mit drei verschiedenen Messvorrichtungen, die in einem Gerät integriert sind. Neben einem Keratometer ist ein Messsystem zur subjektiven Refraktionsbestimmung mit Optotypen vorgesehen. Mittels einer OCT-Messvorrichtung ist eine Bestimmung einer axialen Länge eines Auges möglich. Dabei kann mittels eines Spiegels zwischen einer Messvorrichtung zur objektiven Refraktionsbestimmung und der OCT-Messvorrichtung umgeschaltet werden.

Die DE 10 2017 210577 A1 offenbart ein ophthalmologisches Messgerät zur verbesserten Teilstreckenbestimmung am Auge.

Ein weiteres Messgerät zur Messung einer Vorderkammer des Auges betrifft die EP 1 138 257 A1.

Der vorliegenden Erfindung liegt daher die Aufgabe zugrunde, ein Verfahren und ein Sehprüfsystem vorzuschlagen, mit dem ein Refraktionswert einfach und verlässlich bestimmt werden kann.

Diese Aufgabe wird durch ein Verfahren mit den Merkmalen des Anspruchs 1 sowie durch ein Sehprüfsystem mit den Merkmalen des Anspruchs 10 gelöst.

Das erfindungsgemäße Verfahren zum Überprüfen der Augen eines Probanden wird mit einem Sehprüfsystem durchgeführt, welches eine erste interferometrische Messvorrichtung, eine zweite topografische Messvorrichtung, eine dritte refraktive Messvorrichtung und eine Verarbeitungseinrichtung umfasst, wobei mit der ersten Messvorrichtung eine Achslänge eines Auges des Probanden gemessen wird, wobei mit der zweiten Messvorrichtung eine Krümmung der Hornhaut des Auges gemessen wird, wobei mit der dritten Messvorrichtung eine Refraktionseigenschaft des Auges gemessen wird, wobei an dem Auge eine Messung mit der ersten, zweiten und dritten Messvorrichtung zeitgleich durchgeführt wird, wobei mit der Verarbeitungseinrichtung Messdaten der Messung der ersten, zweiten und dritten Messvorrichtung verarbeitet werden, wobei die Verarbeitungseinrichtung eine Datenbank mit Normaldaten ausweist, wobei als Normaldaten Messdaten von Augen einer Normalpopulation mit einer Achslänge eines Auges, einer Krümmung der Hornhaut des Auges und einer Refraktionseigenschaft des Auges verwendet werden, wobei mittels der Verarbeitungseinrichtung ein Vergleich der Messdaten mit den Normaldaten durchgeführt und ein Ergebnis des Vergleichs ausgegeben wird.

Dadurch, dass die Achslänge des Auges, die Krümmung der Hornhaut des Auges und die objektive Refraktionseigenschaft beziehungsweise ein Refraktionswert des Auges in beispielsweise Dioptrie gleichzeitig gemessen wird, können Messfehler, wie sie bei aufeinanderfolgenden Messungen mit verschiedenen Messgeräten auftreten können, ausgeschlossen werden. Bei der zeitgleichen Messung mit der ersten, zweiten und dritten Messvorrichtung beziehen sich alle gewonnenen Messdaten auf einen zu diesem Zeitpunkt vorliegenden Zustand des Auges, was eine Vergleichbarkeit der Messdaten bei gleichen Bedingungen ermöglicht. Bei der Verwendung unterschiedlicher Geräte oder bei aufeinanderfolgenden Messungen ist das Auge in Folge von Augenbewegungen stets unterschiedlich zu einer Messachse ausgerichtet oder befindet sich in unterschiedlichsten Akkomodationszuständen. Neben der objektiven Bestimmung des Refraktionswerts können bei dem Verfahren Messdaten für Topografie bzw. Krümmung der Hornhaut sowie die Achslänge des Auges ermittelt werden. Aufgrund dieser dann gleichzeitig vorliegenden Messdaten wird es für eine untersuchende Person einfacher möglich den gemessenen Refraktionswert zu beurteilen.

Insbesondere dadurch, dass die Verarbeitungseinrichtung die Datenbank mit den Normaldaten umfasst, und den Vergleich der Messdaten mit den Normaldaten durchführt, kann anhand des Ergebnisses des Vergleichs beziehungsweise der jeweiligen Differenz eines Messwertes mit einem Normalwert eine Abweichung leicht beurteilt werden. Wenn beispielsweise ein Messwert für die Krümmung der Hornhaut von einem Normalwert stark abweicht, kann die untersuchende Person einen ebenfalls vom Normalwert abweichenden Refraktionswert leichter interpretieren. Im vorgenannten Beispiel kann dann die Krümmung der Hornhaut ursächlich für den Refraktionswert sein. Weiter kann eine zu große Achslänge als eine Ursache für einen Refraktionswert von einer untersuchenden Person bestimmt werden. Liegen für die Krümmung der Hornhaut und für die Achslänge des Auges keine von einem Normalwert abweichende Messwerte vor, kann beispielsweise ein Brechungsindex der Linse verändert sein. Insgesamt wird es durch die zeitgleiche Gewinnung der Messwerte möglich einfach und schnell genaue Messwerte zu erhalten und diese mit Normalwerten zu vergleichen. Der Vergleich erfolgt dabei mittels der Verarbeitungseinrichtung, die ein Ergebnis des Vergleichs ausgibt und so einer untersuchenden Person eine einfachere Beurteilung der gewonnenen Messdaten ermöglicht. Die Verarbeitungseinrichtung umfasst dabei Mittel zur Datenverarbeitung und Darstellung, wie beispielsweise einen Computer und einen Bildschirm.

Erfindungsgemäß werden als Normaldaten Messdaten von Augen einer Normalpopulation mit einer Achslänge eines Auges, einer Krümmung der Hornhaut des Auges und einer Refraktionseigenschaft des Auges verwendet. Die Normaldaten können dem fünfzigsten Perzentil einer Vergleichsgruppe von Personen entsprechen an deren Augen die Messdaten ermittelt wurden. Die Normaldaten können auch sämtliche Messdaten der Vergleichsgruppe enthalten, wobei dann die Verarbeitungseinrichtung eine genaue Abweichung der mit dem Sehprüfsystem ermittelten Messdaten vom fünfzigsten Perzentil der Vergleichsgruppe ausgeben kann. Beispielsweise ebenfalls eine Angabe welchem Perzentil der Vergleichsgruppe die Messdaten jeweils zugeordnet werden können. Unter der hier als Normalpopulation bezeichneten Vergleichsgruppe wird ein repräsentativer Bevölkerungsdurchschnitt verstanden.

Mittels der Verarbeitungseinrichtung kann durch den Vergleich der Messdaten mit den Normaldaten eine Bestimmung eines Grades der Refraktion erfolgen. Mit dem Grad der Refraktion kann eine Einstufung der ermittelten Messwerte relativ zu den in der Datenbank enthaltenen Normaldaten anhand der Abweichung von den Normaldaten erfolgen. Ein Grad einer objektiven Refraktion kann anhand der physikalischen Größen der Messwerte von der Verarbeitungseinrichtung ermittelt werden. Dabei kann eine unterschiedliche Gewichtung der Messwerte vorgenommen werden oder die Messwerte können ins Verhältnis gesetzt werden. Der Grad der objektiven Refraktion kann von der Verarbeitungseinrichtung ausgegeben werden, beispielsweise auf einem Bildschirm, sodass eine noch einfachere Beurteilung der Refraktionseigenschaften des Auges möglich wird.

Vorteilhaft kann die Verarbeitungseinrichtung die gemessene Achslänge, die Krümmung und die Refraktionseigenschaft des Auges jeweils mit den Normaldaten der Achslänge, der Krümmung und der Refraktionseigenschaft eines Auges vergleichen, wobei die Verarbeitungseinrichtung die Normaldaten für den Vergleich nach einer Übereinstimmung von Achslänge, Krümmung oder Refraktionseigenschaft mit den Messdaten der Messung auswählen kann. Die Verarbeitungseinrichtung kann demnach nicht nur die jeweiligen Datensätze von Messdaten und Normaldaten für Achslänge, Krümmung und Refraktionseigenschaft unabhängig voneinander miteinander vergleichen, sondern auch aus der Datenbank beziehungsweise den Normaldaten ein Auge beziehungsweise die einem Auge zugehörigen Normaldaten beziehungsweise Messdaten auswählen die dem mit dem Sehprüfsystem gemessenen Messdaten am nächsten kommen beziehungsweise mit diesen übereinstimmen. Die Verarbeitungseinrichtung nimmt demnach einen Vergleich zwischen dem mit dem Sehprüfsystem gemessenen Auge und dem in der Datenbank in Form von Messdaten enthaltenen Augen vor und gibt diesen Vergleich aus. Eine untersuchende Person kann anhand des Vergleichs noch leichter beurteilen, inwieweit das mit dem Sehprüfsystem gemessene Auge von einer Norm abweicht oder bereits bekannte Symptome aufweist.

Die Verarbeitungseinrichtung kann bei dem Vergleich der Messdaten ein Alter des Probanden berücksichtigen. Gleichfalls kann die Datenbank mit den Normaldaten, Normaldaten enthalten, die jeweils einer Altersangabe zugeordnet werden können. Die Verarbeitungseinrichtung kann dann aus der Datenbank mit den Normaldaten die Normaldaten für den Vergleich auswählen, die dem Alter des Probanden entsprechen. Das Alter des Probanden kann beispielsweise über Eingabemittel in die Verarbeitungseinrichtung vor oder nach einer Messung eingegeben werden. Aufgrund der bekannten Abhängigkeit einer Kurzsichtigkeit vom Alter wird durch die Berücksichtigung des Alters ein noch genauerer Vergleich der Messdaten möglich. Darüber hinaus ist es möglich, dass die Verarbeitungseinrichtung bei dem Vergleich der Messdaten beispielsweise eine Prävalenz der Kurzsichtigkeit in der Bevölkerung berücksichtigt. Neben dem Alter können dann auch Tätigkeitsprofile und Dispositionen berücksichtigt werden.

Als Normaldaten können Messdaten des Probanden mit einer Achslänge des Auges, einer Krümmung der Hornhaut des Auges und einer Refraktionseigenschaft des Auges, die zu einem vor der Messung liegenden Zeitpunkt bestimmt wurden, verwendet werden. So ist es möglich mit dem Sehprüfsystem zu unterschiedlichen Zeitpunkten bei ein und demselben Probanden eine Messung vorzunehmen und jeweils Messdaten zu gewinnen und zu speichern. Diese Messdaten können dann als Normaldaten für den Vergleich genutzt werden. So kann eine eventuelle Veränderung der optischen Eigenschaft des betreffenden Auges über einen Zeitabschnitt von beispielsweise Monaten oder Jahren bestimmt werden. Eine Ursache für einen sich beispielsweise verschlechternden Refraktionswert kann so noch einfacher ermittelt werden. Optional ist es aber auch möglich die zu jedem Messzeitpunkt gemessenen Messdaten stets mit Normaldaten einer Vergleichsgruppe zu vergleichen.

Die Verarbeitungseinrichtung kann die Messdaten der dritten Messvorrichtung mit den Messdaten der ersten Messeinrichtung und/oder der zweiten Messeinrichtung korrigieren. Die Bestimmung eines objektiven Refraktionswerts ist häufig schwierig und fehlerbehaftet, da wie vorstehend ausgeführt die Refraktionen von vielen Faktoren und Umgebungsbedingungen abhängig sein kann. Bei der Krümmung der Hornhaut und der Achslänge handelt es sich hingegen um Messwerte, die keinem Einfluss von beispielsweise Medikamenten oder einer Hirnaktivität unterliegen. Die Messdaten der dritten Messvorrichtung können daher mit den Messdaten der ersten und/oder zweiten Messeinrichtung vorteilhaft korrigiert werden.

So kann die Verarbeitungseinrichtung eine Plausibilitätsprüfung für einen gemessenen objektiven Refraktionswert des Auges mit der Achslänge und/oder mit der Krümmung der Hornhaut des Auges durchführen und bei einem abweichenden Refraktionswert den Refraktionswert nach der Achslänge und/oder der Krümmung der Hornhaut korrigieren. Die Plausibilitätsprüfung kann dabei anhand von Bereichsangaben erfolgen, die einen Toleranzbereich für die jeweiligen Messwerte angeben. Die Bereichsangaben können ebenfalls in der Datenbank gespeichert sein. Die Bereichsangaben können auf Erfahrungswerten basieren oder anhand einer Standartabweichung mit statistischen Mitteln bestimmt werden.

Mittels einer Fixationseinrichtung des Sehprüfsystems kann eine für das Auge im Unendlichen fokussierbare Fixationsmarke dargestellt werden, wobei das Auge die Fixationsmarke fokussieren und eine Fixierung des Auges relativ zum Sehprüfsystem erfolgen kann. Wesentlich für eine genaue Messung des Sehprüfsystems ist eine lagegerechte Anordnung des Auges relativ zu einer Messachse des Sehprüfsystems. Vorteilhaft fluchtet dabei eine optische Achse des Auges mit der Messachse des Sehprüfsystems. Die Ausrichtung des Auges kann dabei dadurch erfolgen, dass dem Probanden die Fixationsmarke dargeboten wird, die der Proband im Unendlichen fokussieren kann. Die Fixationsmarke kann beispielsweise eine bildhafte Darstellung eines Gegenstandes sein. Die bilderhafte Darstellung kann durch einen in einen Strahlengang des Sehprüfsystems eingekoppelten Bildschirm erfolgen. Wesentlich ist, dass sich die bildhafte Darstellung für das Auge des Probanden im Unendlichen befindet, sodass der Ziliarmuskel des Auges bei der Messung mit dem Sehprüfsystem vollständig entspannt ist.

Vorteilhaft kann die Überprüfung der Augen dann auch ohne Verabreichung von Zykloplegika durchgeführt werden. Eine Überprüfung der Augen wird dadurch einfacher und schneller möglich.

Das erfindungsgemäße Sehprüfsystem zum Überprüfen der Augen eines Probanden umfasst eine erste interferometrische Messvorrichtung, eine zweite topografische Messvorrichtung, eine dritte refraktive Messvorrichtung und eine Verarbeitungseinrichtung, wobei mit der ersten Messvorrichtung eine Achslänge eines Auges des Probanden messbar ist, wobei mit der zweiten Messvorrichtung eine Krümmung der Hornhaut des Auges messbar ist, wobei mit der dritten Messvorrichtung eine Refraktionseigenschaft des Auges messbar ist, wobei an dem Auge eine Messung mit der ersten, zweiten und dritten Messvorrichtung zeitgleich durchführbar ist, wobei mit der Verarbeitungseinrichtung Messdaten der Messung der ersten, zweiten und dritten Messvorrichtung verarbeitbar sind, wobei die Verarbeitungseinrichtung eine Datenbank mit Normaldaten aufweist, wobei die Normaldaten Messdaten von Augen einer Normalpopulation mit einer Achslänge eines Auges, einer Krümmung der Hornhaut des Auges und einer Refraktionseigenschaft des Auges sind, wobei mittels der Verarbeitungseinrichtung ein Vergleich der Messdaten mit den Normaldaten durchführbar und ein Ergebnis des Vergleichs ausgehbar ist. Zu den Vorteilen des erfindungsgemäßen Sehprüfsystems wird auf die Vorteilsbeschreibung des erfindungsgemäßen Verfahrens verwiesen.

Die erste Messvorrichtung, die zweite Messvorrichtung und die dritte Messvorrichtung können eine gemeinsame Messachse aufweisen, die mit einer optischen Achse des Auges in Übereinstimmung bringbar sein kann. Dadurch das die Messvorrichtungen eine gemeinsame Messachse aufweisen wird eine besonders genaue und gut vergleichbare Gewinnung von Messdaten möglich. Ein Fluchten der gemeinsamen Messachse mit der optischen Achse des Auges ist insbesondere zur genauen Bestimmung einer Achslänge des Auges vorteilhaft.

Die zweite Messvorrichtung und/oder dritte Messvorrichtung kann eine Abstandsmesseinrichtung zur Messung eines Abstandes von Auge und zweiter Messvorrichtung und/oder dritter Messvorrichtung aufweisen. Die Abstandsmesseinrichtung kann den Abstand zwischen der refraktiven Messvorrichtung und/oder der topografischen Messvorrichtung und dem zu untersuchenden Auge messen. Dieser Abstand ist für eine Messdatenkorrektur wesentlich und es wird durch die Gewinnung von Abstandsdaten möglich den Probanden entsprechend korrekt vor dem Sehprüfsystem zu positionieren bzw. die mit der refraktiven Messvorrichtung gemessen Messdaten geeignet zur korrigieren. So kann vorteilhaft der Abstand zwischen der refraktiven Messvorrichtung und der Hornhaut des Auges, insbesondere der Vorderfläche der Hornhaut mit der Abstandsmesseinrichtung messbar sein. Darüber hinaus kann mit der Abstandsmesseinrichtung auch ein Abstand zu der Netzhaut bzw. Hinterfläche des Auges unter Einbeziehung der Achslänge bestimmt werden. Optional ist auch ein Abstand zu einer Hinterfläche der Hornhaut, Vorderfläche der Linse und/oder Hinterfläche der Linse bestimmbar. Die Bauart der Abstandsmesseinrichtung ist prinzipiell beliebig, wobei die Abstandsmesseinrichtung von der topografischen Messvorrichtung, einem Keratometer oder einem Scheimpflugsystem, ausgebildet werden kann.

Die erste Messvorrichtung, zweite Messvorrichtung und/oder dritte Messvorrichtung können eine Fixationseinrichtung zur Fixierung des Auges relativ zum Sehprüfsystem aufweisen. Die Fixationseinrichtung kann beispielsweise einen Bildschirm oder einen geeigneten Projektor umfassen, mit dem eine Fixationsmarke für das Auge im Unendlichen sichtbar darstellbar ist. Die Fixationsmarke kann eine bildhafte Darstellung eines Gegenstands sein, sodass eine Fokussierung des Auges auf einen Punkt vermieden wird. Die Fixationsmarke kann in einem Strahlengang des Sehprüfsystems über beispielsweise einen Teilerwürfel eingekoppelt werden, so dass die Fixationsmarke für den Probanden sichtbar ist.

Die erste Messvorrichtung kann ein Partial Coherence Interferometer (PCI) sein, wobei das Interferometer mit einer kohärenten Lichtquelle, zwei Messarmen und einer Detektoreinrichtung zur gleichzeitigen Erfassung der Vorderfläche der Hornhaut und der Netzhaut beziehungsweise einer optischen Grenzfläche des Auges eingerichtet sein kann. Dadurch, dass das Interferometer über zwei Messarme verfügt, ist es möglich die Vorderfläche des Auges und die Netzhaut gleichzeitig zu detektieren und einen Relativabstand von Vorderfläche und Netzhaut und damit die Achslänge des Auges zu bestimmen. Dabei ist es unwesentlich, in welchem Abstand sich das Auge relativ zu dem Sehprüfsystem befindet, da die Abstandsmessung beziehungsweise Messung der Achslänge des Auges unabhängig zu dem Abstand vom Sehprüfsystem mit dem Interferometer durchgeführt werden kann. Eventuelle Messfehler des Interferometers aufgrund einer Abstandslage des Auges relativ zum Sehprüfsystem können so gänzlich ausgeschlossen werden. Die Achslänge wird damit besonders genau messbar. Neben der Achslänge können noch weitere optische Grenzflächen des Auges, wie Hinterfläche der Hornhaut, Vorderfläche der Linse, Hinterfläche der Linse und Abstände zwischen den optischen Grenzfläche mit dem Interferometer gemessen und von der Verarbeitungseinrichtung verarbeitet werden. Diese Messdaten können ebenfalls von der Verarbeitungseinrichtung für einen Vergleich mit entsprechenden Normaldaten herangezogen werden.

Die zweite Messvorrichtung kann einen Keratometer und/oder Scheimpflugsystem sein. Mit derartigen Messvorrichtungen ist es möglich eine Topografie bzw. die Krümmung der Hornhaut des Auges zu bestimmen.

Das Keratometer kann eine Beobachtungseinrichtung mit einer Kamera und mit der Kamera erfassbare Messmarken, die von einem kreisförmigen, nicht kollimierten Leuchtstreifen und zwei kollimierten Leuchtpunkten ausgebildet sein können, aufweisen. Eine Bauart der Messmarken des Keratometers ist grundsätzlich beliebig, wobei als Leuchtmittel Leuchtdioden vorgesehen werden können. Der Leuchtstreifen kann von einem kreisförmigen Lichtleiterelement erzeugt werden. Auch kann vorgesehen sein eine Mehrzahl konzentrischer, ringförmiger Leuchtstreifen zu erzeugen. Vorteilhaft kann als Licht für die Messmarken Infrarotlicht verwendet werden. Die Beobachtungseinrichtung kann eine Kamera sein, die über einen Teilerwürfel in einen Strahlengang des Sehprüfsystems bzw. des Keratometers eingekoppelt ist. Mit der Kamera kann ein Reflexbild der Messmarken auf der Hornhaut des Auges erfasst werden. Mittels Bildverarbeitung kann aus dem Reflexbild der Messmarken einfach eine Krümmung der Hornhaut abgeleitet und von der Verarbeitungseinrichtung dargestellt werden. Weiter ist es möglich die Kamera als Einrichtkamera bzw. Übersichtskamera für das Sehprüfsystem zur lagegenauen Anordnung und Ausrichtung des Auges des Probanden zu nutzen.

Das Scheimpflugsystem kann eine Projektionseinrichtung, die zur Beleuchtung des Auges mit einem Lichtspalt eingerichtet sein kann, und eine Beobachtungseinrichtung mit einer Kamera, welche zur Aufnahme eines Schnittbilds des Lichtspalts im Auge eingerichtet sein kann, aufweisen, wobei die Projektionseinrichtung und die Kamera nach der Scheimpflugregel relativ zueinander angeordnet sein können. Mit der Projektionseinrichtung kann dann der Lichtspalt auf das Auge projiziert werden, sodass eine Spaltbeleuchtung des Auges entlang der optischen Achse des Auges beziehungsweise der Sehachse erfolgen kann. Mit der nach dem Scheimpflugprinzip angeordneten Kamera kann das so erzeugte Schnittbild des Lichtspalts in dem Auge aufgenommen werden, sodass ein beleuchteter Querschnittsbereichs des Auges beziehungsweise ein vorderer Abschnitts des Auges optisch erfassbar sein kann. Ein sich so ergebenes Längsschnittbild des Auges kann dann vorzugsweise die optischen Grenzflächen der Hornhaut und der Linse wiedergeben. Aus einem so erhaltenen Bilddatensatz kann die Verarbeitungseinrichtung die Relativabstände der optischen Grenzflächen einfach berechnen. Weiter kann auch die Krümmung der Hornhaut einfach ermittelt werden. Das Scheimpflugsystem kann alleine oder auch zusammen mit dem Keratometer die zweite Messvorrichtung ausbilden.

Vorteilhaft kann die dritte Messvorrichtung ein Autorefraktometer sein.

Das Autorefraktometer kann eine Projektionseinrichtung, die zur Projektion eines Beleuchtungsmusters auf die Netzhaut des Auges eingerichtet sein kann, und eine Beobachtungseinrichtung, mit einer Beugungseinheit und einer Kamera, welche zur Aufnahme des Beleuchtungsmusters im Auge eingerichtet sein kann, aufweisen. Die Projektion des Beleuchtungsmusters kann dabei so erfolgen, dass das Beleuchtungsmuster auf der Netzhaut fokussiert ist. Mit der optischen Beobachtungseinrichtung kann das an der Netzhaut reflektierte Beleuchtungsmuster durch die Linse des Auges hindurch betrachtet werden, sodass ein Abbildungsmuster auf einem fotoelektrischen Sensor der Kamera abgebildet wird. Dieses Abbildungsmuster wird von der Kamera aufgenommen und mittels Bildbearbeitung ausgewertet. Entsprechend der Refraktionseigenschaften des Auges ist das auf die Netzhaut projizierte Beleuchtungsmuster charakteristisch verzerrt, sodass im Rahmen der Auswertung des Beleuchtungsmusters aus einem Maß der Verzerrung die Refraktionseigenschaften des Auges abgeleitet werden können. Die Beugungseinheit kann beispielsweise eine Lochblende mit einer Anzahl kreisförmig angeordneter Löcher sein, wobei über ein Ablenkprisma oder eine entsprechende Linse die Strahlengänge der Löcher jeweils auf den Sensor der Kamera abgelenkt werden können. Alternativ kann die Beugungseinheit auch ein diffraktives optisches Element (DOE) sein.

Weitere vorteilhafte Ausführungsformen eines Sehprüfsystems ergeben sich aus den Merkmalsbeschreibungen der auf den Verfahrensanspruch 1 rückbezogenen Unteransprüche.

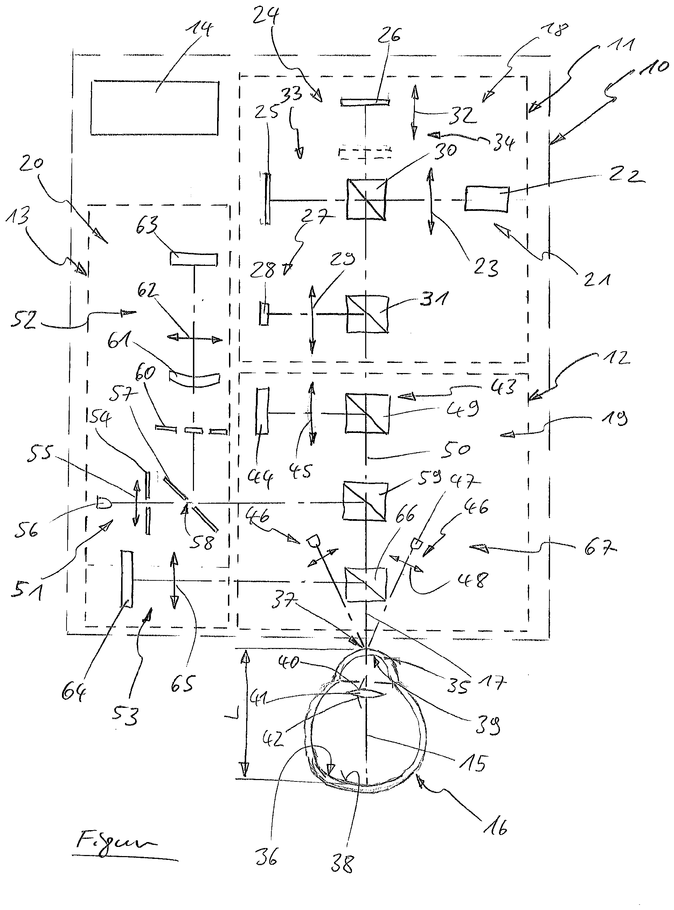

Im Folgenden wird die Erfindung oder Bezugnahme auf die beigefügte Zeichnung näher erläutert.

Die **Figur** zeigt eine schematische Darstellung eines Aufbaus eines Sehprüfsystems 10 umfassend eine erste interferometrische Messvorrichtung 11, eine zweite topografische Messvorrichtung 12, eine dritte refraktive Messvorrichtung 13 und eine Verarbeitungseinrichtung 14. Das Sehprüfsystem 10 ist relativ zu einer Sehachse 15 bzw. optische Achse eines zu untersuchenden Auges 16 so angeordnet, dass die Sehachse 15 mit einer Messachse 17 des Sehprüfsystems 10 übereinstimmt. Die erste interferometrische Messvorrichtung 11 wird von einem Partial Coherence Interferometer 18, die zweite topografische Messvorrichtung 12 von einem Keratometer 19 und die dritte refraktive Messvorrichtung 13 von einem Autorefraktometer 20 ausgebildet.

Das Interferometer 18 ist im Wesentlichen aus einer Lasereinrichtung 21 mit einer Laserlichtquelle 22 und einer Linsenanordnung 23 , einer Spieleinrichtung 24 mit einem ersten Spiegel 25 und einem zweiten Spiegel 26, einer Detektoreinrichtung 27 mit einem Detektor 28 und einer Linsenanordnung 29 sowie einem ersten Teilerwürfel 30 und einem zweiten Teilerwürfel 31 ausgebildet. Insbesondere der zweite Spiegel 26 ist längs verschieblich entlang des Doppelpfeils 32 angeordnet, sodass eine Länge eines zweiten Referenzarms 34 beziehungsweise einer entsprechenden Referenzstrecke veränderlich ist. Ein erster Referenzarm 33 ist hingegen nicht längenveränderlich ausgebildet. Durch eine Verschiebung des zweiten Spiegels 26 können verschiedene, auf der Sehachse 15 liegende Bereiche des Auges 16 abgetastet werden. Insbesondere ist es möglich eine Achslänge (L) des Auges 16 von der Hornhaut 35 bis zu der Netzhaut 36 beziehungsweise von einer Vorderfläche 37 der Hornhaut 35 bis zu einer Hinterfläche 38 der Netzhaut 36 zu messen. Auf eine weitergehende Erläuterung einer bekannten Funktion des Partial Coherence Interferometer 18 wird hier verzichtet. Weiter können auch Messdaten gewonnen werden, die Relativpositionen optischer Grenzflächen auf der Sehachse 15, wie die Vorderfläche 37 der Hornhaut 35, der Hinterfläche 39 der Hornhaut 35, eine Vorderfläche 40 einer Linse 41, einer Hinterfläche 42 der Linse 41 und die Hinterfläche 38 der Netzhaut 36, beschreiben.

Das Keratometer 19 umfasst eine Beobachtungseinrichtung 43 mit einer Kamera 44 und einer Linsenanordnung 45 sowie mit der Kamera 44 erfassbare Messmarken 46, die jeweils von einer Infrarotlichtquelle 47 und einer Linsenanordnung 48 ausgebildet sind. Die Infrarotlichtquelle kann beispielsweise eine Leuchtdiode sein. Die Messmarken 46 können zwei kollimierte Leuchtpunkte auf der Hornhaut 35 ausbilden, die mit der Kamera 44 erfassbar sind. Die Messmarken 46 sind hier mit einem kreisförmigen, nicht kollimierten Leuchtstreifen, der nicht dargestellt ist, ergänzt. Die Beobachtungseinrichtung 43 ist über einen Teilerwürfel 49 in einen Strahlengang 50 des Sehprüfsystems 10 eingekoppelt.

Das Autorefraktometer 20 dient zur Bestimmung der Refraktionseigenschaften des Auges 16 und umfasst im Wesentlichen eine Projektionseinrichtung 51 und eine Beobachtungseinrichtung 52 sowie eine Fixationseinrichtung 53. Mit der optischen Projektionseinrichtung 51 kann ein Beleuchtungsmuster auf die Netzhaut 36 des Auges 16 projiziert und dort fokussiert werden. Die Projektionseinrichtung 51 umfasst dabei eine Lochblende 54, eine Linsenanordnung 55 und eine Infrarotlichtquelle 56. Das Beleuchtungsmuster wird durch einen Spiegel 57 mit einer Lochblende 58 hindurch über einen ersten Teilerwürfel 59 des Autorefraktometers 20 in den Strahlengang 50 des Sehprüfsystems eingekoppelt. Die optische Beobachtungseinrichtung 52 umfasst eine 6-fach Lochblende 60, ein Ablenkprisma 61, eine Linsenanordnung 62 und eine Kamera 63. Die mit der Kamera 63 aufgenommenen Bilddaten werden in der Verarbeitungseinrichtung 14 verarbeitet und ausgewertet, um die Refraktionseigenschaften des Auges 16 zu bestimmen. Die Beobachtungseinrichtung 52 ist über den Spiegel 57 und den ersten Teilerwürfel 59 in den Strahlengang 50 eingekoppelt.

Die Fixationseinrichtung 53 des Autorefraktometers 20 ist aus einem Bildschirm 64 zur bildhaften Darstellung einer Fixationsmarke und einer Linsenanordnung 65 zur Darstellung der Fixationsmarke im Unendlichen ausgebildet. Ein zweiter Teilerwürfel 66 ermöglicht die Einkopplung der Fixationsmarke in den Strahlengang 50. Der erste Teilerwürfel 59 und der zweite Teilerwürfel 66 sind Bestandteil des Autorefraktometers 20.

Weiter ist eine Abstandsmesseinrichtung 67 vorgesehen, die hier von dem Keratometer 19 ausgebildet ist. Die Abstandsmesseinrichtung 67 umfasst die Messmarken 46 und die Beobachtungseinrichtung 43.

Bei dem hier dargestellten Sehprüfsystem 10 erfolgt eine zeitgleiche Messung mit der ersten Messvorrichtung 11, der zweiten Messvorrichtung 12 und der dritten Messvorrichtung 13, wobei mit der Verarbeitungseinrichtung 14 Messdaten der Messung der ersten Messvorrichtung 11, der zweiten Messvorrichtung 12 und der dritten Messvorrichtung 13 verarbeitet werden, wobei die Verarbeitungseinrichtung 14 eine hier nicht dargestellte Datenbank mit Normaldaten aufweist, wobei mittels der Verarbeitungseinrichtung 14 ein Vergleich der Messdaten mit den Normaldaten durchgeführt und ein Ergebnis des Vergleichs ausgegeben wird.

## Patentansprüche

1. Verfahren zum Überprüfen der Augen eines Probanden mit einem Sehprüfsystem (10), umfassend eine erste Messvorrichtung (11), eine zweite topografische Messvorrichtung (12), eine dritte refraktive Messvorrichtung (13) und eine Verarbeitungseinrichtung (14), wobei mit der ersten Messvorrichtung eine Achslänge (L) eines Auges (16) des Probanden gemessen wird, wobei mit der zweiten Messvorrichtung eine Krümmung der Hornhaut (35) des Auges gemessen wird, wobei mit der dritten Messvorrichtung eine Refraktionseigenschaft des Auges gemessen wird, wobei mit der Verarbeitungseinrichtung Messdaten der Messung der ersten, zweiten und dritten Messvorrichtung verarbeitet werden,
**dadurch gekennzeichnet,**
**dass** an dem Auge eine Messung mit der ersten, zweiten und dritten Messvorrichtung zeitgleich durchgeführt wird, wobei die Verarbeitungseinrichtung eine Datenbank mit Normaldaten aufweist, wobei als Normaldaten Messdaten von Augen einer Normalpopulation mit einer Achslänge eines Auges, einer Krümmung der Hornhaut des Auges und einer Refraktionseigenschaft des Auges verwendet werden, wobei mittels der Verarbeitungseinrichtung ein Vergleich der Messdaten mit den Normaldaten durchgeführt und ein Ergebnis des Vergleichs ausgegeben wird.

2. Verfahren nach Anspruch 1,
**dadurch gekennzeichnet,**
**dass** mittels der Verarbeitungseinrichtung (14) durch den Vergleich der Messdaten mit den Normaldaten eine Bestimmung eines Grades der Refraktion erfolgt.

3. Verfahren nach Anspruch 1 oder 2,
**dadurch gekennzeichnet,**
**dass** die Verarbeitungseinrichtung (14) die gemessene Achslänge (L), die Krümmung und die Refraktionseigenschaft des Auges (16) jeweils mit den Normaldaten der Achslänge (L), der Krümmung und der Refraktionseigenschaft eines Auges vergleicht, wobei die Verarbeitungseinrichtung die Normaldaten für den Vergleich nach einer Übereinstimmung von Achslänge (L), Krümmung oder Refraktionseigenschaft mit den Messdaten der Messung auswählt.

4. Verfahren nach einem der vorangehenden Ansprüche,
**dadurch gekennzeichnet,**
**dass** die Verarbeitungseinrichtung (14) bei dem Vergleich der Messdaten ein Alter des Probanden berücksichtigt.

5. Verfahren nach einem der vorangehenden Ansprüche,
**dadurch gekennzeichnet,**
**dass** als Normaldaten Messdaten des Probanden mit einer Achslänge (L) des Auges (16), einer Krümmung der Hornhaut (35) des Auges und einer Refraktionseigenschaft des Auges, die zu einem vor der Messung liegenden Zeitpunkt bestimmt wurden, verwendet werden.

6. Verfahren nach einem der vorangehenden Ansprüche,
**dadurch gekennzeichnet,**
**dass** die Verarbeitungseinrichtung (14) die Messdaten der dritten Messvorrichtung (13) mit den Messdaten der ersten Messvorrichtung (11) und/oder der zweiten Messvorrichtung (12) korrigiert.

7. Verfahren nach Anspruch 6,
**dadurch gekennzeichnet,**
**dass** die Verarbeitungseinrichtung (14) eine Plausibilitätsprüfung für einen gemessenen objektiven Refraktionswert des Auges (16) mit der Achslänge (L) und/oder mit der Krümmung der Hornhaut (35) des Auges durchführt und bei einem abweichenden Refraktionswert den Refraktionswert nach der Achslänge (L) und/oder der Krümmung der Hornhaut korrigiert.

8. Verfahren nach einem der vorangehenden Ansprüche,
**dadurch gekennzeichnet,**
**dass** mittels einer Fixationseinrichtung (53) des Sehprüfsystems (10) eine für das Auge (16) im Unendlichen fokussierbare Fixationsmarke dargestellt wird, wobei das Auge die Fixationsmarke fokussiert und eine Fixierung des Auges relativ zum Sehprüfsystem erfolgt.

9. Verfahren nach einem der vorangehenden Ansprüche,
**dadurch gekennzeichnet,**
**dass** die Überprüfung der Augen (16) ohne Verabreichung von Zykloplegika durchgeführt wird.

10. Sehprüfsystem (10) zum Überprüfen der Augen eines Probanden, umfassend eine erste Messvorrichtung (11), eine zweite topografische Messvorrichtung (12), eine dritte refraktive Messvorrichtung (13) und eine Verarbeitungseinrichtung (14), wobei mit der ersten Messvorrichtung eine Achslänge (L) eines Auges (16) des Probanden messbar ist, wobei mit der zweiten Messvorrichtung eine Krümmung der Hornhaut (35) des Auges messbar ist, wobei mit der dritten Messvorrichtung eine Refraktionseigenschaft des Auges messbar ist, wobei mit der Verarbeitungseinrichtung Messdaten der Messung der ersten, zweiten und dritten Messvorrichtung verarbeitbar sind, wobei die erste, zweite und dritte Messvorrichtung in einem Gerät integriert sind,
**dadurch gekennzeichnet,**
**dass** an dem Auge eine Messung mit der ersten, zweiten und dritten Messvorrichtung zeitgleich durchführbar ist, wobei die Verarbeitungseinrichtung eine Datenbank mit Normaldaten aufweist, wobei die Normaldaten Messdaten von Augen einer Normalpopulation mit einer Achslänge eines Auges, einer Krümmung der Hornhaut des Auges und einer Refraktionseigenschaft des Auges sind, wobei mittels der Verarbeitungseinrichtung ein Vergleich der Messdaten mit den Normaldaten durchführbar und ein Ergebnis des Vergleichs ausgebbar ist.

11. Sehprüfsystem nach Anspruch 10,
**dadurch gekennzeichnet,**
**dass** die erste Messvorrichtung (11), die zweite Messvorrichtung (12) und die dritte Messvorrichtung (13) eine gemeinsame Messachse (17) aufweisen, die mit der optischen Achse (15) des Auges (16) in Übereinstimmung bringbar ist.

12. Sehprüfsystem nach Anspruch 10 oder 11,
**dadurch gekennzeichnet,**
**dass** die zweite Messvorrichtung (12) und/oder dritte Messvorrichtung (13) eine Abstandsmesseinrichtung (67) zur Messung eines Abstandes von Auge (16) und zweiter Messvorrichtung und/oder dritter Messvorrichtung aufweist.

13. Sehprüfsystem nach einem der Ansprüche 10 bis 12,
**dadurch gekennzeichnet,**
**dass** die erste Messvorrichtung (11), zweite Messvorrichtung (12) und/oder dritte Messvorrichtung (13) eine Fixationseinrichtung (53) zur Fixierung des Auges (16) relativ zum Sehprüfsystem (10) aufweist.

14. Sehprüfsystem nach einem der Ansprüche 10 bis 13,
**dadurch gekennzeichnet,**
**dass** die erste Messvorrichtung (11) eine Ultraschall-Messvorrichtung ist.

15. Sehprüfsystem nach einem der Ansprüche 10 bis 13,
**dadurch gekennzeichnet,**
**dass** die erste Messvorrichtung (11) eine interferometrische Messvorrichtung ist.

16. Sehprüfsystem nach Anspruch 15,
**dadurch gekennzeichnet,**
**dass** die erste Messvorrichtung (11) ein Interferometer zur optischen Kohärenzinterferometrie (OCT) ist.

17. Sehprüfsystem nach Anspruch 15,
**dadurch gekennzeichnet,**
**dass** die erste Messvorrichtung (11) ein Partial Coherence Interferometer (18) ist, wobei das Interferometer mit einer kohärenten Lichtquelle (22), zwei Messarmen (33, 34) und einer Detektoreinrichtung (27) zur gleichzeitigen Erfassung der Vorderfläche (37) und der Netzhaut (36) des Auges (16) eingerichtet ist.

18. Sehprüfsystem nach einem der Ansprüche 10 bis 17,
**dadurch gekennzeichnet,**
**dass** die zweite Messvorrichtung (12) ein Keratometer (19) und/oder ein Scheimpflugsystem ist.

19. Sehprüfsystem nach Anspruch 18,
**dadurch gekennzeichnet,**
**dass** das Keratometer (19) eine Beobachtungseinrichtung (43) mit einer Kamera (44) und mit der Kamera erfassbare Messmarken (46), die von einem kreisförmigen, nicht kollimierten Leuchtstreifen und zwei kollimierten Leuchtpunkten ausgebildet sind, aufweist.

20. Sehprüfsystem nach Anspruch 18 oder 19,
**dadurch gekennzeichnet,**
**dass** das Scheimpflugsystem eine Projektionseinrichtung, die zur Beleuchtung des Auges mit einem Lichtspalt eingerichtet ist, und eine Beobachtungseinrichtung mit einer Kamera, welche zur Aufnahme eines Schittbildes des Lichtspalts im Auge (16) eingerichtet ist, aufweist, wobei die Projektionseinrichtung und die Kamera nach der Scheimpflugregel relativ zueinander angeordnet sind.

21. Sehprüfsystem nach einem der Ansprüche 10 bis 20,
**dadurch gekennzeichnet,**
**dass** die dritte Messvorrichtung (13) ein Autorefraktometer (20) ist.

22. Sehprüfsystem nach Anspruch 21,
**dadurch gekennzeichnet,**
**dass** das Autorefraktometer (20) eine Projektionseinrichtung (51), die zur Projektion eines Beleuchtungsmusters auf die Netzhaut (36) des Auges (16) eingerichtet ist, und eine Beobachtungseinrichtung (52), mit einer Beugungseinheit (60, 61) und einer Kamera (63), welche zur Aufnahme des Beleuchtungsmusters im Auge eingerichtet ist, aufweist.

## Claims

1. A method for testing the eyes of a test person with the aid of a vision testing system (10), comprising a first measuring device (11), a second topographic measuring device (12), a third refractive measuring device (13) and a processing means (14), an axial length (L) of an eye (16) of the test person being measured with the aid of the first measuring device, a curvature of the cornea (35) of the eye being measured with the aid of the second measuring device, a refractive property of the eye being measured with the aid of the third measuring device, measurement data of the measurements of the first, second and third measuring device being processed with the aid of the processing means,
**characterised in that**
a measurement is simultaneously carried out with the first, second and third measuring device at the eye, said processing means presenting a database with normal data, measurement data of eyes of a normal population, with an axial length of an eye, a curvature of the cornea of the eye and a refractive property of the eye, being used as the normal data, a comparison of the measurement data with the normal data being carried out and a result of said comparison being issued by means of said processing means.

2. The method according to claim 1,
**characterised in that**
a degree of the refraction is determined by means of the processing means (14) by way of the comparison of the measurement data with the normal data.

3. The method according to claim 1 or 2,
**characterised in that**
the processing means (14) in each instance compares the measured axial length (L), the curvature and the refractive property of the eye (16) with the normal data of the axial length (L), the curvature and the refractive property of an eye, said processing means selecting the normal data for the comparison according to a consistency of the axial length (L), the curvature or the refractive property with the measurement data of the measurement.

4. The method according to any one of the preceding claims,
**characterised in that**
the processing means (14) takes an age of the test person into account when comparing the measurement data.

5. The method according to any one of the preceding claims,
**characterised in that**
measurement data of the test person that were determined at a point of time before the measurement, with an axial length (L) of the eye (16), a curvature of the cornea (35) of the eye and a refractive property of the eye, are used as normal data.

6. The method according to any one of the preceding claims,
**characterised in that**
the processing means (14) corrects the measurement data of the third measuring device (13) with the aid of the measurement data of the first measuring device (11) and/or of the second measuring device (12).

7. The method according to claim 6,
**characterised in that**
the processing means (14) carries out a plausibility check for a measured objective refraction value of the eye (16) with the axial length (L) and/or with the curvature of the cornea (35) of the eye and corrects, when the refraction value differs, said refraction value according to the axial length (L) and/or the curvature of the cornea.

8. The method according to any one of the preceding claims,
**characterised in that**
a fixation mark that can be focused by the eye (16) at infinity is displayed by means of a fixation means (53) of the vision testing system (10), the eye focusing on the fixation mark and the eye being fixed in relation to the vision testing system.

9. The method according to any one of the preceding claims,
**characterised in that**
the eyes (16) are tested without administering cycloplegic agents.

10. A vision testing system (10) for testing the eyes of a test person, comprising a first measuring device (11), a second topographic measuring device (12), a third refractive measuring device (13) and a processing means (14), an axial length (L) of an eye (16) of the test person being measured with the aid of the first measuring device, a curvature of the cornea (35) of the eye being measured with the aid of the second measuring device, a refractive property of the eye being measured with the aid of the third measuring device, said processing means being configured to process measurement data of the measurements of the first, second and third measuring device, said first, second and third measuring device being integrated in one piece of equipment,
**characterised in that**
the first, second and third measuring device are configured to simultaneously carry out a measurement at the eye, the processing means presenting a database with normal data, measurement data of eyes of a normal population, with an axial length of an eye, a curvature of the cornea of the eye and a refractive property of the eye, being used as the normal data, said processing means being configured to carry out a comparison of the measurement data with the normal data and to issue a result of said comparison.

11. The vision testing system according to claim 10,
**characterised in that**
the first measuring device (11), the second measuring device (12) and the third measuring device (13) present a common measurement axis (17) that can be harmonised with the optical axis (15) of the eye (16).

12. The vision testing system according to claim 10 or 11,
**characterised in that**
the second measuring device (12) and/or the third measuring device (13) present/s a distance measurement means (67) for measuring a distance between the eye (16) and the second measuring device and/or third measuring device.

13. The vision testing system according to any one of the claims 10 to 12,
**characterised in that**
the first measuring device (11), the second measuring device (12) and/or the third measuring device (13) present/s a fixation means (53) for fixing the eye (16) in relation to the vision testing system (10).

14. The vision testing system according to any one of the claims 10 to 13,
**characterised in that**
the first measuring device (11) is an ultrasonic measuring device.

15. The vision testing system according to any one of the claims 10 to 13,
**characterised in that**
the first measuring device (11) is an interferometric measuring device.

16. The vision testing system according to claim 15,
**characterised in that**
the first measuring device (11) is an interferometer for optical coherence interferometry (OCT).

17. The vision testing system according to claim 15,
**characterised in that**
the first measuring device (11) is a partial coherence interferometer (18), said interferometer being designed to have a coherent light source (22), two measuring arms (33, 34) and a detector means (27) for simultaneously capturing the front face (37) and the retina (36) of the eye (16).

18. The vision testing system according to any one of the claims 10 to 17,
**characterised in that**
the second measuring device (12) is a keratometer (19) and/or a Scheimpflug system.

19. The vision testing system according to claim 18,
**characterised in that**
the keratometer (19) presents an examination means (43) having a camera (44) and measuring marks (46) that can be captured with the aid of the camera and that are realised by a fluorescent strip that is circular and not collimated and by two collimated luminous spots.

20. The vision testing system according to claim 18 or 19,
**characterised in that**
the Scheimpflug system presents a projection means that is designed for illuminating the eye with a light gap and an examination means having a camera that is designed for capturing a sectional view of the light gap in the eye (16), said projection means and the camera being disposed in relation to each other according to the Scheimpflug principle.

21. The vision testing system according to any one of the claims 10 to 20,
**characterised in that**
the third measuring device (13) is an autorefractometer (20).

22. The vision testing system according to claim 21,
**characterised in that**
the autorefractometer (20) presents a projection means (51) that is designed for projecting a lighting pattern onto the retina (36) of the eye (16) and an examination means (52), having a diffraction unit (60, 61) and a camera (63) that is designed for capturing the lighting pattern in the eye.

## Revendications

1. Procédé destiné au contrôle des yeux d'une personne examinée à l'aide d'un système d'examen de la vue (10), comprenant un premier dispositif de mesure (11), un deuxième dispositif de mesure topographique (12), un troisième dispositif de mesure réfractif (13) et une unité de traitement (14), une longueur axiale (L) d'un oeil (16) de la personne examinée étant mesurée à l'aide du premier dispositif de mesure, une courbure de la cornée (35) de l'oeil étant mesurée à l'aide du deuxième dispositif de mesure, une propriété de réfraction de l'oeil étant mesurée à l'aide du troisième dispositif de mesure, des données mesurées provenant de la mesure du premier, deuxième et troisième dispositif de mesure étant traitées à l'aide de l'unité de traitement,
**caractérisé en ce que**
les mesures de l'oeil sont relevées à l'aide du premier, deuxième et troisième dispositif de mesure en même temps, l'unité de traitement présentant une base de données comportant des données normales, des données mesurées des yeux d'une population normale, comportant une longueur axiale d'un oeil, une courbure de la cornée de l'oeil et une propriété de réfraction de l'oeil étant utilisées comme données normales, l'unité de traitement comparant les données mesurées avec les données normales et émettant un résultat de la comparaison.

2. Procédé selon la revendication 1,
**caractérisé en ce que**
l'unité de traitement (14) détermine un degré de la réfraction en comparant des données mesurées avec les données normales.

3. Procédé selon la revendication 1 ou 2,
**caractérisé en ce que**
l'unité de traitement (14) compare la longueur axiale (L) mesurée, la courbure et la propriété de réfraction de l'oeil (16) avec les données normales de la longueur axiale (L), de la courbure et de la propriété de réfraction d'un oeil, l'unité de traitement choisissant les données normales pour la comparaison selon une cohérence de la longueur axiale (L), la courbure ou la propriété de réfraction avec les données mesurées de la mesure.

4. Procédé selon l'une quelconque des revendications précédentes, **caractérisé en ce que**
l'unité de traitement (14) prend un âge de la personne examinée en compte dans la comparaison des données mesurées.

5. Procédé selon l'une quelconque des revendications précédentes, **caractérisé en ce que**
des données mesurées de la personne examinée sont utilisées comme données normales, lesdites données mesurées comportant une longueur axiale (L) de l'oeil (16), une courbure de la cornée (35) de l'oeil et une propriété de réfraction de l'oeil qui sont déterminées avant la mesure.

6. Procédé selon l'une quelconque des revendications précédentes, **caractérisé en ce que**
l'unité de traitement (14) corrige les données mesurées du troisième dispositif de mesure (13) avec les données mesurées du premier dispositif de mesure (11) et/ou deuxième dispositif de mesure (12).

7. Procédé selon la revendication 6,
**caractérisé en ce que**
l'unité de traitement (14) conduit un contrôle de plausibilité pour une valeur de réfraction objective mesurée de l'oeil (16) avec la longueur axiale (L) et/ou avec la courbure de la cornée (35) de l'oeil et corrige la valeur de réfraction selon la longueur axiale (L) et/ou la courbure de la cornée si la valeur de réfraction diffère.

8. Procédé selon l'une quelconque des revendications précédentes, **caractérisé en ce**
**qu'**une unité de fixation (53) du système d'examen de la vue (10) affiche une marque de fixation qui peut être focalisée par l'oeil (16) à l'infini, l'oeil focalisant la marque de fixation et étant fixé par rapport au système d'examen de la vue.

9. Procédé selon l'une quelconque des revendications précédentes, **caractérisé en ce que**
les yeux (16) sont contrôlés sans administrant des cycloplégiques.

10. Système d'examen de la vue (10) destiné au contrôle des yeux d'une personne examinée, ledit système d'examen de la vue comprenant un premier dispositif de mesure (11), un deuxième dispositif de mesure topographique (12), un troisième dispositif de mesure réfractif (13) et une unité de traitement (14), une longueur axiale (L) d'un oeil (16) de la personne examinée pouvant être mesurée à l'aide du premier dispositif de mesure, une courbure de la cornée (35) de l'oeil pouvant être mesurée à l'aide du deuxième dispositif de mesure, une propriété de réfraction de l'oeil pouvant être mesurée à l'aide du troisième dispositif de mesure, des données mesurées provenant de la mesure du premier, deuxième et troisième dispositif de mesure pouvant être traitées à l'aide de l'unité de traitement, le premier, deuxième et troisième dispositif de mesure étant intégré dans un appareil, **caractérisé en ce que**
les mesures de l'oeil sont relevées à l'aide du premier, deuxième et troisième dispositif de mesure en même temps, l'unité de traitement présentant une base de données comportant des données normales, les données normales étant des données mesurées des yeux d'une population normale, comportant une longueur axiale d'un oeil, une courbure de la cornée de l'oeil et une propriété de réfraction de l'oeil, l'unité de traitement pouvant comparer les données mesurées avec les données normales et pouvant émettre un résultat de la comparaison.

11. Système d'examen de la vue selon la revendication 10,
**caractérisé en ce que**
le premier dispositif de mesure (11), le deuxième dispositif de mesure (12) et le troisième dispositif de mesure (13) présentant un axe de mesure (17) commun qui peut être harmonisé avec l'axe optique (15) de l'œil (16).

12. Système d'examen de la vue selon la revendication 10 ou 11, **caractérisé en ce que**
le deuxième dispositif de mesure (12) et/ou le troisième dispositif de mesure (13) présente/nt une unité de mesure de la distance (67) pour mesurer la distance entre l'oeil (16) et le deuxième dispositif de mesure et/ou le troisième dispositif de mesure.

13. Système d'examen de la vue selon l'une quelconque des revendications 10 à 12,
**caractérisé en ce que**
le premier dispositif de mesure (11), le deuxième dispositif de mesure (12) et/ou le troisième dispositif de mesure (13) présente/nt une unité de fixation (53) pour fixer l'oeil (16) par rapport au système d'examen de la vue (10).

14. Système d'examen de la vue selon l'une quelconque des revendications 10 à 13,
**caractérisé en ce que**
le premier dispositif de mesure (11) est un dispositif de mesure par ultrasons.

15. Système d'examen de la vue selon l'une quelconque des revendications 10 à 13,
**caractérisé en ce que**
le premier dispositif de mesure (11) est un dispositif de mesure interférométrique.

16. Système d'examen de la vue selon la revendication 15,
**caractérisé en ce que**
le premier dispositif de mesure (11) est un interféromètre pour l'interférométrie à cohérence optique (OCT).

17. Système d'examen de la vue selon la revendication 15,
**caractérisé en ce que**
le premier dispositif de mesure (11) est un interféromètre à cohérence partielle (18), ledit interféromètre comportant une source lumineuse cohérente (22), deux bras de mesure (33, 34) et une unité détecteur (27) pour examiner en même temps la face antérieure (37) et la rétine (36) de l'oeil (16).

18. Système d'examen de la vue selon l'une quelconque des revendications 10 à 17,
**caractérisé en ce que**
le deuxième dispositif de mesure (12) est un kératomètre (19) et/ou un système de Scheimpflug.

19. Système d'examen de la vue selon la revendication 18,
**caractérisé en ce que**
le kératomètre (19) présente une unité d'observation (43) comportant une caméra (44) et des marques de mesure (46) qui peuvent être saisies par la caméra, et qui sont réalisées par une bande lumineuse circulaire non collimatée et deux points lumineux collimatés.

20. Système d'examen de la vue selon la revendication 18 ou 19,
**caractérisé en ce que**
le système de Scheimpflug présente une unité de projection qui est configurée pour illuminer l'oeil à l'aide d'une fente de lumière et une unité d'observation comportant une caméra, ladite unité d'observation étant configurée pour acquérir une image en coupe de la fente de lumière dans l'oeil (16), ladite unité de projection et la caméra étant disposées l'une par rapport à l'autre selon la loi de Scheimpflug.

21. Système d'examen de la vue selon l'une quelconque des revendications 10 à 20,
**caractérisé en ce que**
le troisième dispositif de mesure (13) est un autoréfractomètre (20).

22. Système d'examen de la vue selon la revendication 21,
**caractérisé en ce que**
l'autoréfractomètre (20) présente une unité de projection (51) qui est configurée pour projeter un motif d'éclairage sur la rétine (36) de l'oeil (16) et une unité d'observation (52) comportant une unité de diffraction (60, 61) et une caméra (63) qui est configurée pour imager le motif d'éclairage dans l'oeil.
